# EUROPÄISCHE PATENTANMELDUNG

(11) **EP 4 427 765 A1**
(43) Veröffentlichungstag der Anmeldung: **11.09.2024**
(21) Anmeldenummer: 24162098.8
(22) Anmeldetag: 07.03.2024
(51) Int. Cl.: A61L 2/07, A61B 90/70, A61L 2/18, G16H 40/00, B65B 55/02, A61B 50/30

(54) **VERFAHREN ZUM HANDHABEN VON AUFBEREITUNGSFÄHIGEM, MEDIZINISCHEM STERILGUT**

(30) Priorität: 10.03.2023 DE 102023106039
(71) Anmelder: Aesculap AG, 78532 Tuttlingen (DE)
(72) Erfinder: GOERZ, Dennis, 78532 Tuttlingen (DE); THOMAS, Stefan, 78532 Tuttlingen (DE)
(74) Vertreter: Winter, Brandl - Partnerschaft mbB

(57) **Zusammenfassung**

Die vorliegende Offenbarung betrifft ein Verfahren zur Bereitstellung, Entsorgung und Aufbereitung von medizinischem Sterilgut, insbesondere von chirurgischen Instrumenten, das die getrennte Entsorgung von Sterilgut beinhaltet, das am Ort der medizinischen Versorgung einem Sterilbarrieresystem entnommenem wurde. Die getrennte Entsorgung erfolgt dabei in Abhängigkeit von der potentiellen Kontamination des Sterilgutes am Ort der medizinischen Versorgung. In Folge können Teilprozesse der Aufbereitung in Abhängigkeit der unterschiedlichen, potentiellen Kontamination der beiden Gruppen von Sterilgut unterschiedlich appliziert werden, was Potential zur Effizienzsteigerung im Sterilgutkreislauf birgt.

## Beschreibung

### Technisches Gebiet

Die vorliegende Offenbarung betrifft ein Verfahren zur Bereitstellung, Entsorgung und Aufbereitung von medizinischem Sterilgut.

### Hintergrund der Offenbarung

Ein Sterilgutkreislauf von aufbereitungsfähigem Sterilgut in der medizinischen Versorgung umfasst die Bereitstellung, Entsorgung, Aufbereitung und erneute Bereitstellung des Sterilgutes, insbesondere von medizinischen Instrumenten und Produkten. Neben höchsten Qualitäts- und Hygieneanforderungen besteht fortwährend die Anforderung, die Effizienz des Sterilgutkreislaufs zu steigern. Aus diesem Grund, und um das Bedienpersonal in der Aufbereitung zu entlasten, ist es üblich, Reinigungs-, Desinfektions- und Sterilisationsprozesse automatisiert auszuführen. Andere Tätigkeiten hingegen, insbesondere in der Vorreinigung und beim Packen und Kommissionieren, sind manuell oder weisen zumindest teilweise manuelle Tätigkeiten auf.

### Stand der Technik

Ein Teil des chirurgischen Instrumentariums ist in "Grundsieben" strukturiert, die so gestaltet sind, dass eine möglichst große Anzahl von ähnlichen chirurgischen Indikationen mit einem "Siebtitel" abgedeckt werden kann. Ein "Grundsieb" ist allgemein ein Metallbehältnis, in der Regel eine flache, rechteckige, verschließbare Metallschale mit einem siebartig perforierten Boden, in dem die allgemeinchirurgischen Instrumente gelagert werden, die bei einer Operation gebraucht werden können. Das Instrumentensieb stellt somit quasi die Grundausstattung des Operateurs dar. Es enthält eine Vielzahl von beispielsweise Scheren, Haken, Klemmen, Pinzetten und Zangen, Zubehör für Wundspreizer, Elektrokauter und anderes mehr. Unter dem Begriff "Siebtitel" versteht man allgemein jene medizinische Fachabteilung oder Disziplin (beispielsweise "herzchirurgische Abteilung" oder dergleichen Fakultät), welcher eine Anzahl von Grundsieben zugeordnet ist. Diese Grundsieb-Systematik hat sich etabliert, da sie eine Kompromisslösung bezüglich Wirtschaftlichkeit und Platzbedarf im Vergleich zu einzelindikationsbezogenen Siebtiteln darstellt. Systematisch bringt sie aber mit sich, dass pro Operation nur ein Teil des Instrumentariums zum Einsatz kommt. Die Erfahrung zeigt, dass der Anteil des eingesetzten Instrumentariums pro OP im Durchschnitt durchaus kleiner als etwa 30% sein kann, was auch durch Studien wie "Surgical instrumentation: the true cost of instrument trays and a potential strategy for optimization" Journal of Hospital Administration 2015, Vol. 4, No. 6 gestützt wird.

Ein typischer Sterilgutkreislauf zur Bereitstellung, Entsorgung und Aufbereitung von chirurgischen Instrumenten durchläuft den Ort der medizinischen Versorgung, beispielsweise den OP-Saal und die Aufbereitungseinheit für medizinische Produkte, die AEMP. Grundlegend gilt hierbei der Standard, dass alle einer Sterilbarriere entnommenen (oder entzogenen) Instrumente als "kontaminiert" anzusehen sind und bezüglich Reinigung, Desinfektion und Sterilisation identisch zu behandeln sind, unabhängig davon, ob sie benutzt wurden, oder nicht. In anderen Worten ausgedrückt, ist erst einmal ein Sterilbehälter geöffnet, gelten alle darin sich befindenden Instrumente als kontaminiert unabhängig davon, ob sie dem Sterilbehälter entnommen wurden oder nicht. Ergänzend wird in den USA in der Regel eine sogenannte "three-sink method" vor der maschinellen Aufbereitung angewendet, bei der jedes Instrument einer manuellen Vorreinigung unterzogen wird.

Grundsätzlich durchläuft also nicht eingesetztes Instrumentarium den gleichen aufwendigen Prozess wie eingesetztes Instrumentarium, obwohl es aufgrund des Verschmutzungsgrads nicht zwingend notwendig wäre. Es werden somit unnötige Handhabungsschritte durchgeführt, die unnötigen Personaleinsatz und somit entsprechende Kosten erzeugen. Setzt man den oben genannten Nutzungsgrad von etwa 30% an, durchlaufen somit 70% des Instrumentariums unnötige, manuelle Handhabungsschritte.

### Kurzbeschreibung der Offenbarung

Demgegenüber ist eine Aufgabe der vorliegenden Offenbarung, ein Verfahren zur Bereitstellung, Entsorgung und Aufbereitung von medizinischem Sterilgut in der medizinischen Versorgung bereitzustellen, das einen effizienteren Sterilgutkreislauf ermöglicht.

Die Aufgabe wird gelöst durch ein Verfahren mit den Merkmalen des Anspruchs 1. Vorteilhafte Weiterbildungen sind Teil der Unteransprüche, welche ggf. auch gesondert beanspruchbar sein sollen.

Konkret wird die Aufgabe bevorzugt durch ein Verfahren zur Bereitstellung und Aufbereitung von aufbereitungsfähigem Sterilgut in der medizinischen Versorgung gelöst, das zumindest folgende Schritte aufweist:
- Einen Schritt "Bereitstellen eines Sterilbarrieresystems (auch als Sterilraum bzw. Sterilraumkonzept zu bezeichnen) mit darin enthaltenem Sterilgut an einem Ort der medizinischen Versorgung", vorzugsweise in einem OP. Das Sterilgut ist dabei vorzugsweise eine Menge chirurgischer Instrumente, Arbeitsgerätschaften oder anderweitiger medizinischer Gegenstände, die gemäß einer zu behandelnden, medizinischen Indikation auf einem Sterilgutträger, vorzugsweise einem Siebträger (oder Siebkorb), zusammengestellt ist. Im Folgenden wird der Einfachheit halber von Instrumenten als Sterilgut gesprochen. Diese können einteilig oder mehrteilig, insbesondere gelenkig, beispielsweise als Ringgriffinstrument (wie Schere, Zange, Klemme, etc.) ausgeführt sein. Das Sterilbarrieresystem ist vorzugsweise ein Sterilgutcontainer, der zuvor innerhalb einer Aufbereitungseinheit für medizinische Produkte - im Folgenden AEMP - mit seinem (Sieb-)Titel entsprechend zusammengestelltem Sterilgut / Anzahl von Instrumenten bestückt wurde. Die Instrumente sind hierbei innerhalb des Sterilbarrieresystems/Sterilgutcontainers auf einem Bereitstellungsträger, vorzugsweise dem Siebträger, angeordnet.
- Einen Schritt "Separieren des mit den Instrumenten bestückten Bereitstellungsträgers/Siebträgers (Siebkorbs) vom Sterilbarrieresystem (Sterilgutcontainer)", sodass die Instrumente dem in der medizinischen Versorgung tätigen Personal zugänglich gemacht werden.
- Einen Schritt "Bereitstellen eines separat zum wenigstens einen Bereitstellungsträger (18) vorgesehenen Entsorgungsträgers". Hierbei wird mindestens ein weiterer Siebträger (Siebkorb) bereitgestellt. Bei größeren Instrumentensets auf dem Bereitstellungsträger kann es auch vorteilhaft sein, mehrere Entsorgungsträger (Siebkörbe) bereitzustellen. Dieser Schritt kann auch zu einem früheren oder späteren Zeitpunkt im Verfahrensablauf durchgeführt werden. Sofern mehrere Entsorgungsträger (Siebkörbe) bereitgestellt werden, kann dieser Schritt auch in Teilschritte aufgeteilt werden, die zu unterschiedlichen Zeitpunkten durchgeführt werden. Entscheidend ist, dass mindestens ein Entsorgungsträger (Siebkorb) bereitgestellt ist, wenn der Schritt "Platzieren des entnommenen Sterilgutes (46, 47) auf dem wenigstens einen Entsorgungsträger (19)" durchgeführt wird.
- Einen Schritt "Entnehmen von Instrumenten vom Bereitstellungsträger/Siebträger (Siebkorb)" vorzugsweise durch medizinisches Personal oder robotergestützt. Hierbei werden die entnommenen Instrumente vorzugsweise auf einem Bereitstellungsplatz, insbesondere auf einer Instrumentenablage, insbesondere auf einem Instrumententisch abgelegt, und insbesondere vorgehalten, von dem sich dann das chirurgische Personal die benötigten Instrumente entnimmt.
   In der medizinischen Praxis ist von hier ausgehend ein medizinischer Eingriff möglich oder beabsichtigt, wobei manche der entnommenen und vorgehaltenen Instrumente - im Grenzfall alle - ggf. nicht für den Eingriff verwendet werden müssen, also lediglich vorgehalten bleiben. Alle entnommenen Instrumente gelten jedoch als "benutzt" und damit als kontaminiert.
   Für die gesamte Offenbarung gilt, dass dieser in der Praxis mögliche oder beabsichtigte medizinische Eingriff und die medizinische Versorgung mittels des entnommenen Sterilgutes und der entnommenen Instrumente und deren Verwendung am Patienten, keinen Teil und keinen Schritt des offenbarungsmäßigen Verfahrens darstellen. Dementsprechend sind der medizinische Eingriff/die medizinische Versorgung und die Verwendung der Instrumente am Patienten kein Teil der Erfindung und nicht erfindungswesentlich.
- Einen Schritt "Platzieren des entnommenen Sterilgutes (46, 47) auf dem wenigstens einen Entsorgungsträger (19)" vorzugsweise durch medizinisches Personal oder robotergestützt. In anderen Worten: die Instrumente werden nach ihrer Entnahme vom Bereitstellungsträger (also aktiv benutzt) nicht mehr mit den dort verbliebenen (also nicht aktiv benutzten) Instrumenten gemischt. Durch diese einfache Maßnahme der Trennung (aktiv) entnommener Instrumente von nicht entnommenen (aber als kontaminiert geltenden) Instrumenten ermöglicht das Verfahren, die beiden so definierten Instrumentengruppen im OP oder in der AEMP zumindest abschnittsweise einer unterschiedlichen Aufbereitung zuzuführen. Insbesondere müssen die nicht (aktiv) entnommenen Instrumente nicht zwingend alle Schritte des Aufbereitungsprozesses in vollem Umfang durchlaufen. So ist ein effizienteres Verfahren zur Handhabe von aufbereitungsfähigem Sterilgut bereitgestellt.
- Einen Schritt "Belassen des nicht entnommenen Sterilgutes auf dem wenigstens einen Bereitstellungsträger (18)". Dieser Schritt bedeutet, dass über das gesamte Verfahren hinweg keine Vermischung von entnommenem und nicht entnommenem Sterilgut erfolgt, indem z.B. zum Abschluss einer Operation das nicht entnommene Sterilgut vom Bereitstellungsträger in den Entsorgungsträger überführt wird. Dies wird gemäß dem Stand der Technik teilweise gemacht, um für Transportvorgänge des Sterilgutes nach einer Operation weniger Platz auf einem Transportwagen zu benötigen oder um eine bessere Stapelbarkeit von Siebträgern (Sterilkörben) zu erreichen.
- Es folgt ein Schritt "Entsorgen der entnommenen und der nicht entnommenen (insgesamt als kontaminiert zu bezeichnenden) Instrumente". Die Entsorgung erfolgt vorzugsweise durch eine Sammlung der Instrumente und deren gesicherte Verbringung zur AEMP.
- Bevorzugt in der AEMP erfolgt ein Schritt "Aufbereiten der entsorgten - das heißt sowohl der entnommenen/benutzten als auch der nicht entnommenen/unbenutzten - Instrumente". Dieser Schritt kann auch teilweise im oder am OP erfolgen.
- Einen Schritt, in dem ein besonders arbeitsintensiver Aufbereitungsschritt im OP oder bevorzugt in der AEMP, insbesondere ein manueller oder zumindest teilmanueller Aufbereitungsschritt, vorzugsweise die manuelle Vorreinigung der Instrumente, allein auf den Entsorgungsträger und die darauf angeordneten Instrumente angewendet wird, und nicht auf den Bereitstellungsträger und dessen Instrumente. Auf diese Weise durchlaufen, wie weiter oben dargelegt, etwa 70% weniger Instrumente die manuelle Vorreinigung, was eine erhebliche Einsparung manueller Arbeit darstellt.
- Einen weiteren Schritt, in dem der Bereitstellungsträger direkt einem Reinigungs- und Desinfektionsgerät zugeführt wird, wohingegen der Inhalt des Entsorgungsträgers - in üblicher Art und Weise - zunächst vorgereinigt wird. Die Bearbeitung im Reinigungs- und Desinfektionsgerät erfordert dabei keine manuelle oder teilmanuelle Bearbeitung.

In einer Weiterbildung des Verfahrens wird ein Zurücklegen von entnommenen Instrumenten in den Bereitstellungsträger durch eine Maßnahme und/oder Vorrichtung (Sperre) unterbunden (bzw. verhindert).

Gemäß einer bevorzugten Weiterbildung hat das Verfahren einen Schritt "Vorhalten der entnommenen Instrumente auf einem Bereitstellungsplatz, insbesondere auf einer Instrumentenablage, insbesondere auf einem Instrumententisch".

Besonders effizient erweist sich das Verfahren, wenn auch der Bereitstellungsträger im Anschluss an die medizinische Versorgung zur Entsorgung genutzt wird. Dies erfolgt einfach derart, dass die darauf befindlichen, nicht entnommenen Instrumente dort verbleiben und der Bereitstellungträger der AEMP (separat) zugeführt wird.

Die AEMP wird mit dem Bereitstellungsträger und dem Entsorgungsträger beschickt. Vorzugsweise erfolgt die Beschickung mittels schonendem (vibrationslosem oder wenigsten vibrationsarmem bzw. rüttelfreien oder wenigsten rüttelarmem) Transport, derart, dass eine insbesondere auf dem Bereitstellungträger vorhandene Ordnung (Ausrichtung und/oder Lage) der nicht entnommenen Instrumente erhalten bleibt.

Grundlegend ist festzuhalten, dass natürlich mehr als ein Bereitstellungsträger und, falls nötig, mehr als ein Entsorgungsträger vorgesehen sein/werden können.

Vorzugsweise sind/werden wenigstens zwei Bereitstellungsträger vorgesehen, wenn zum einen einteilige Instrumente und zum anderen mehrteilige oder gelenkige Instrumente bereitzustellen sind. Da diese sich in der Aufbereitung in der AEMP, insbesondere in ihrer Wartung, unterscheiden können, ist es von Vorteil, sie allein aus diesem Grund bereits getrennt der AEMP zuführen zu können. Entsprechend ist es von Vorteil, für die unterschiedlichen Arten von Wartung einen separaten Entsorgungsträger vorzusehen.

Gemäß einer bevorzugten Weiterbildung wird ein Bereitstellungsträger im Sterilbarrieresystem mit wartungsbedürftigen und/oder mehrteiligen Instrumenten bestückt bereitgestellt, die bereits in wartungsfähiger Position und/oder Lage am Bereitstellungsträger angeordnet sind/werden. Diese wartungsfähige Anordnung ermöglicht den Durchlauf der nicht entnommenen Instrumente, ohne dass sie bei jedem Durchlauf von ihrem Platz am Bereitstellungsträger entnommen werden müssen. Es entfällt so ein erheblicher Bestückungs- und Platzierungsaufwand.

Gemäß einer bevorzugten Weiterbildung wird ein Bereitstellungsträger im Sterilbarrieresystem mit wartungsfreien (z.B. ohne interne Funktion in Form von Lagern, Gelenken, Motoren, etc.) und/oder einteiligen Instrumenten bestückt bereitgestellt.

Unabhängig davon, ob die Instrumente wartungsbedürftig und/oder mehrteilig oder wartungsfrei und/oder einteilig sind, ist es von Vorteil, wenn gemäß einer Weiterbildung der Bereitstellungsträger so mit den Instrumenten bestückt bereitgestellt wird, dass diese sich in einer vorbestimmten, und insbesondere reproduzierbaren, Ordnung und Lage befinden.

Vorzugsweise beinhaltet der Schritt "Aufbereiten" einen Schritt "Reinigen und Desinfizieren" mittels einer Reinigungs- und Desinfektionseinrichtung (RDG) der AEMP und einen Schritt "Sterilisieren" mittels einer Sterilisationseinrichtung.

Vorzugsweise beinhaltet der Schritt "Aufbereiten der entnommenen und der nicht entnommenen Instrumente" wenigstens einen der Schritte "Kontrollieren der Instrumente" und "Warten der Instrumente, insbesondere von deren Gelenken, insbesondere mittels Ölen und/oder Bewegen".

Vorzugsweise beinhaltet der Schritt "Aufbereiten der entnommenen und der nicht entnommenen Instrumente" einen Schritt "Bestücken des Bereitstellungsträgers mit den zumindest vorgereinigten, entnommenen Instrumenten". In anderen Worten: Haben die Instrumente des Entsorgungsträgers die Vorreinigung durchlaufen, weisen sie nominell den gleichen Status auf, wie die nicht entnommenen Instrumente des Bereitstellungsträgers. Sie sind frei von groben Verschmutzungen, und könnten, falls vorteilhaft, die Folgeprozesse RDG und Sterilisation gemeinsam mit den noch auf dem Bereitstellungsträger befindlichen Instrumenten durchlaufen.

Gemäß einer bevorzugten Weiterbildung erfolgt der jeweilige Schritt "Kontrollieren", "Warten" oder "Bestücken" frühestens nach dem manuellen oder zumindest teilmanuellen Aufbereitungsschritt, insbesondere der Vorreinigung, besonders bevorzugt aber nach der Reinigung und Desinfektion im RDG.

Die offenbarungsgemäße Vorgehensweise, nicht entnommene und entnommene Instrumente der AEMP auf getrennten Trägern - Bereitstellungsträger und Entsorgungsträger - zuzuführen, beinhaltet die Möglichkeit, die anderen Aufbereitungsschritte, beispielsweise die "Reinigung und Desinfektion" und die "Sterilisation" - oder nur einen davon - in Bezug auf wenigstens einen Prozessparameter des jeweiligen Aufbereitungsschrittes unterschiedlich anzuwenden. So können beispielsweise die nicht entnommenen und daher als unbenutzt klassifizierten Instrumente des Bereitstellungsträgers einem darauf angepassten und nachhaltigeren Aufbereitungsprozess unterzogen werden, als die als benutzt klassifizierten Instrumente des Entsorgungsträgers. Genannt werden können hier als Prozessparameter eine kürzere Laufzeit im RDG, der Einsatz von "sanfteren" Chemikalien oder geringerer Mengen von Chemikalien.

Alternativ können die Träger natürlich wenigstens einen der Schritte "Reinigung und Desinfektion" und "Sterilisation", oder beide, mit identischen Prozessparametern durchlaufen.

Prinzipiell kann der Schritt "Bestücken des Bereitstellungsträgers mit den entnommenen und vorgereinigten Instrumenten" vor der Reinigung und Desinfektion erfolgen, vorzugsweise erfolgt er jedoch nach diesem Schritt, jedoch spätestens vor der Sterilisation.

Um die Zusammengehörigkeit eines Entsorgungsträgers zu dem Bereitstellungsträger, von dem die Instrumente entnommen wurden, zu kennzeichnen, wird gemäß einer Weiterbildung - vor einem Beladen des Sterilbarrieresystems mit dem Bereitstellungsträger - am Bereitstellungsträger ein lesbarer, auslesbarer und/oder scanbarer Identifikator angeordnet. Zudem wird dem Bereitstellungsträger ein lesbarer, auslesbarer oder scanbarer Identifikator beigelegt, der nach dem Separieren des Bereitstellungsträgers vom Sterilbarrieresystem entnommen wird und an einem Entsorgungsträger angeordnet wird, welcher bevorzugt unabhängig vom Sterilbarrieresystem bereitgestellt wird. Die Identifikatoren sind vorzugsweise leicht manuell als auch maschinell lesbar und insbesondere auf einfache Weise, vorzugsweise mittels Verrastung oder Clip, am Entsorgungsträger befestigbar. Eine Zuordnung wird vorzugsweise durch gleiche Farbgebung und/oder Beschriftung der einander zugeordneten Identifikatoren erleichtert. Natürlich kann einem Entsorgungsträger eine Vielzahl von Identifikatoren zugeordnet sein, sollten die entnommenen Instrumente auf verschiedene Entsorgungsträger verteilt werden.

In einer Weiterbildung werden die Identifikatoren zum Bündeln von Bereitstellungsträgern und/oder von Entsorgungsträgern genutzt, wofür sie vorzugsweise als Verrastungs- oder Clipverbinder ausgebildet sind. Bei Bereitstellung können so vorzugsweise mehrere, zusammengehörige Bereitstellungsträger zusammen aus dem Sterilbarrieresystem entnommen werden. Die Identifikatoren sind konstruktiv vorzugsweise als Klickschilder ausgeführt und in ihrer Höhe so angepasst, dass ihre Koppelabschnitte in entsprechende Koppelabschnitte der gestapelten Bereitstellungsträger einrasten.

Im Anschluss an die Reinigung und Desinfektion im RDG werden der/die Bereitstellungsträger und der/die Entsorgungssiebkörbe vorzugsweise an einen Bestückungs- oder Packplatz gestellt. Für das Nachfolgende gibt es verschiedene mögliche, weitere Verfahrensschritte:
A) Die Instrumente des Entsorgungsträgers werden auf Sauberkeit und Beschädigungen kontrolliert und an für sie vorbestimmten Lagerplätzen am Bereitstellungsträger platziert, bzw. bestückt und Ringgriffinstrumente werden in ihrer Lagerposition gewartet, insbesondere zumindest geölt.

In erster Alternative findet kein Bewegen der Ringgriffinstrumente statt, um das Öl zu verteilen.

In zweiter Alternative wird der Bereitstellungsträger, sollte er Ringgriffinstrumente enthalten, mechanisch, vorzugsweise über eine Rüttelplatte, in Bewegung versetzt, sodass sich das Öl verteilt.

In dritter Alternative wird am Packplatz erfasst, wie häufig der mit dem Identifikator versehene Bereitstellungsträger - und damit insbesondere die darauf befindlichen, nicht entnommenen Instrumente - aufbereitet wurde und ein Instrumenten Management System zeigt dem Personal nach einer definierten Anzahl von Aufbereitungszyklen, beispielsweise jedes zehnte Mal, an, dass die Instrumente bewegt werden müssen, was gegebenenfalls erfolgt. Bevorzugt werden die Instrumente hierzu einzeln oder in Gruppen entnommen.
B) Die Instrumente des Entsorgungsträgers werden auf Sauberkeit und Beschädigungen kontrolliert und an für sie vorbestimmten Lagerplätzen am Bereitstellungsträger platziert, bzw. bestückt und Ringgriffinstrumente werden in ihrer Lagerposition geölt, und danach einzeln oder in Gruppen bewegt. Bevorzugt werden die Instrumente hierzu einzeln oder in Gruppen entnommen und anschließend zurück an ihren Lagerplatz platziert, bzw. bestückt.
C) Am Bereitstellungsträger angeordnete Ringgriffinstrumente werden in ihrer Lagerposition geölt, danach einzeln oder in Gruppen entnommen, bewegt und zurück an ihren Lagerplatz platziert, bzw. bestückt. Im Entsorgungsträger angeordnete Instrumente werden auf Sauberkeit und Beschädigungen kontrolliert. Darunter befindliche Ringgriffinstrumente werden geölt und bevorzugt bewegt. Die Instrumente des Entsorgungsträgers werden an vorbestimmten Lagerplätzen in den Bereitstellungsträger platziert, bzw. bestückt.
D) Am Bereitstellungsträger angeordnete Ringgriffinstrumente werden in ihrer Lagerposition geölt. Sollte der Bereitstellungsträger Ringgriffinstrumente aufweisen, wird er mechanisch, vorzugsweise über eine Rüttelplatte, in Bewegung versetzt, sodass sich das Öl verteilt. Am Entsorgungsträger angeordnete Instrumente werden auf Sauberkeit und Beschädigungen kontrolliert. Darunter befindliche Ringgriffinstrumente werden geölt und bevorzugt bewegt. Die Instrumente des Entsorgungsträgers werden an vorbestimmten Lagerplätzen in den Bereitstellungsträger platziert, bzw. bestückt.
E) Am Bereitstellungsträger angeordnete Ringgriffinstrumente werden in ihrer Lagerposition geölt. Es wird am Packplatz erfasst, wie häufig der mit dem Identifikator versehene Bereitstellungsträger - und damit insbesondere die darauf befindlichen, nicht entnommenen Instrumente - aufbereitet wurde und ein Instrumenten Management System zeigt dem Personal nach einer definierten Anzahl von Aufbereitungszyklen, beispielsweise jedes zehnte Mal, an, dass die Instrumente bewegt werden müssen, was gegebenenfalls erfolgt. Bevorzugt werden die Instrumente hierzu einzeln oder in
Gruppen entnommen. Am Entsorgungsträger angeordnete Instrumente werden auf Sauberkeit und Beschädigungen kontrolliert. Darunter befindliche Ringgriffinstrumente werden geölt und bevorzugt bewegt. Die Instrumente des Entsorgungsträgers werden an vorbestimmten Lagerplätzen in den Bereitstellungsträger, platziert bzw. bestückt.

Das offenbarungsgemäße Verfahren bietet den Vorteil, manuelle Aufwände, den notwendigen Mitarbeitereinsatz und Kosten reduzieren zu können, wobei gleichzeitig ein größerer Durchsatz bei gleichem Mitarbeitereinsatz erfolgen kann. Zudem können nicht entnommene/unbenutzte Instrumente mit auf ihren geringeren Verschmutzungsgrad angepassten, nachhaltigeren Aufbereitungsprozessen, beispielsweise mit kürzeren Laufzeiten der RDGs, dem Einsatz von "sanfteren" Chemikalien und dergleichen, aufbereitet werden. Unbenutzte Instrumente können siebträgerweise gehandhabt werden, was die Möglichkeit eröffnet, diesen Prozesszweig vollautomatisiert aufzubauen/umzusetzen. Ein Vorsortieren am Pack- oder Bestückungsplatz entfällt, da dieser Schritt nur notwendig ist, damit nach einer definierten Pack- oder Bestückungsreihenfolge gepackt/bestückt werden kann. Die Einhaltung der Reihenfolge ist im neuen Verfahren allerdings nicht mehr notwendig. Nach Kontrolle eines Instrumentes kann dieses direkt an den vorgesehenen Lagerplatz im Bereitstellungsträger platziert, bzw. bestückt werden. Da die benutzten Instrumente getrennt von den unbenutzten Instrumenten an den Pack- oder Bestückungsplatz gelangen, kann im Rahmen eines Rücklagerungsprozesses erfasst werden, beispielsweise mittels Scannen eines Datamatrix-Codes, welche Instrumente des entsprechenden Bereitstellungsträgers bei welcher OP im Einsatz waren und welche nicht. Bei Erfassung dieser Daten über einen längeren Zeitraum lassen sich statistische Auswertungen durchführen, anhand derer beispielsweise Bereitstellungsträger-Zusammenstellungen oder Siebtitel für die entsprechende Indikation optimiert oder der Nutzungsgrad von Instrumenten erhöht werden kann.

Auch ist es möglich, basierend auf den so erfassten Daten individuelle Pflege- und/oder Wartungsarbeiten auszulösen. So können beispielsweise auch predictive maintenance Tätigkeiten ausgelöst werden. Beispielsweise kann für eine Schere angezeigt werden, dass diese noch x Mal aufbereitet werden kann, bevor sie voraussichtlich geschärft werden muss. Oder es kann angezeigt werden, dass ein individuelles Instrument oder y Instrumente (absolut oder prozentuale Angabe) innerhalb der nächsten z Aufbereitungszyklen gewartet (z.B. geölt) werden muss/müssen. Auf diese Weise kann sich der Mitarbeiter überlegen, ob er diesen Wartungsschritt vorzieht, wenn es seine Arbeitsbelastung erlaubt.

### Kurzbeschreibung der Figuren

Fig. 1 zeigt eine schematische Darstellung eines Sterilgutkreislaufs vom OP über eine AEMP zurück zum OP und darauf abgebildet Schritte eines Verfahrens zur Handhabe von aufbereitungsfähigem Sterilgut, gemäß der vorliegenden Offenbarung.
Fig. 2 zeigt einen geschlossenen Bereitstellungsträger zur Verwendung im Verfahren gemäß Figur 1, in perspektivischer Ansicht,
Fig. 3 zeigt gestapelte und verbundene Bereitstellungsträger zur Verwendung im Verfahren gemäß Figur 1, in perspektivischer Ansicht,
Fig. 4 zeigt gestapelte, bestückte Bereitstellungsträger in einem geöffneten Sterilgutcontainer zur Verwendung im Verfahren gemäß Figur 1, in perspektivischer Ansicht,
Fig. 5 zeigt den oberen der Bereitstellungsträger gemäß Figur 4, der mit Ringgriff instrumenten bestückt ist, in perspektivischer Ansicht,
Fig. 6 zeigt den unteren der Bereitstellungsträger gemäß Figur 4, der mit einteiligen Instrumenten bestückt ist, in perspektivischer Ansicht,
Fig. 7 zeigt einen Entsorgungsträger zur Verwendung im Verfahren gemäß Figur 1, in perspektivischer Ansicht, und
Fig. 8 zeigt das Verfahren zur Handhabe von aufbereitungsfähigem Sterilgut gemäß Figur 1, gemäß der vorliegenden Offenbarung, in detaillierter Darstellung.

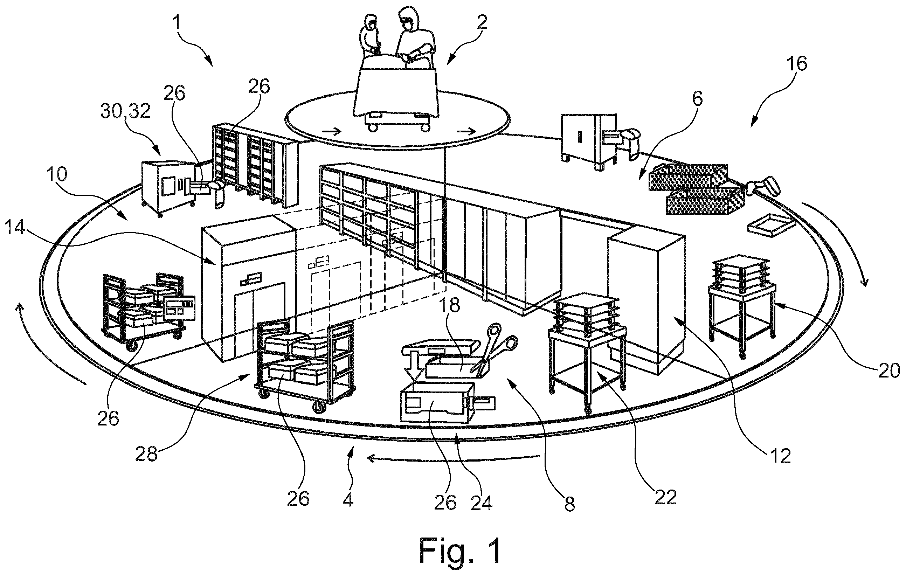

Figur 1 zeigt eine schematische Darstellung eines Sterilgutkreislaufs 1, symbolisiert durch die umlaufenden Pfeile, vom OP 2 über eine AEMP 4 zurück zum OP 2, sowie darauf abgebildet, Schritte eines Verfahrens zur Handhabe von aufbereitungsfähigem Sterilgut, gemäß der vorliegenden Offenbarung. Die AEMP ist aufgeteilt in eine Unreinseite 6, eine Reinseite 8 und eine Sterilseite 10. Zwischen der Unreinseite und der Reinseite sind Reinigungs- und Desinfektionsgeräte 12 angeordnet, zwischen der Reinseite und der Sterilseite sind Sterilisatoren 14 angeordnet.

Der Sterilgutkreislauf 1 hat folgende Abschnitte:
Auf der Unreinseite 6 befindet sich ein Wareneingang 16, an dem aus dem OP entsorgtes, kontaminiertes Sterilgut, im Folgenden als Instrumente bezeichnet, entgegengenommen und registriert wird; sowie eine manuelle Aufbereitung (nicht dargestellt), in der eine Vorreinigung von als kontaminiert registrierten Instrumenten erfolgt; sowie eine Beladung (Beladestation) 20 der Reinigungs- und Desinfektionsgeräte (RDGs) 12 mit allen eingegangenen Instrumenten.

Auf der Reinseite, am Ausgang der RDGs 12, erfolgt eine Freigabe 22 der gereinigten Instrumente; von dieser werden die Instrumente an einem Pack- oder Bestückungsplatz 24 übergeben; dort werden die auf verschiedenen Bereitstellungs- und Entsorgungsträgern gereinigten und desinfizierten Instrumente auf einem oder auf mehreren Bereitstellungsträgern 18 zusammengeführt. Der jeweilige Bereitstellungsträger 18 ist dann mit den für die Indikation benötigten Instrumenten bestückt. Am Pack- oder Bestückungsplatz 24 erfolgt auch die oben beschriebene Wartung gemäß den Varianten A bis E und zuletzt das Verpacken des oder der bestückten Bereitstellungsträger(s) 18 in ein Sterilbarrieresystem 26, insbesondere einen Sterilgutcontainer 26. Sofern an dem Entsorgungsträger 19 ein Identifikator vorgesehen war, um dessen Zuordnung zu einem Bereitstellungsträger 18 sicher zu stellen, wird dieser Identifikator zurück in bzw. an den Bereitstellungsträger 18 verbracht und die Zuordnung des Entsorgungsträgers 19 zum Bereitstellungsträger 18 aufgehoben. Es folgt die reinseitige Beladung 28 der Sterilisatoren (Autoklaven) 14 mit den Sterilgutcontainern 26.

Auf der Sterilseite 10 erfolgt wiederum eine Freigabe 30 und in Folge eine Kommissionierung und Lagerung 32 der Sterilgutcontainer 26.

Dem OP werden in Folge in Abhängigkeit der Indikation die entsprechend der/des "Siebtitel(s)" benötigten Sterilgutcontainer 26, mit den darin enthaltenen, bestückten Bereitstellungsträgern, bereitgestellt. Entsorgungsträger 19 werden dem OP unabhängig von den benötigten Sterilgutcontainern 26 bereitgestellt.

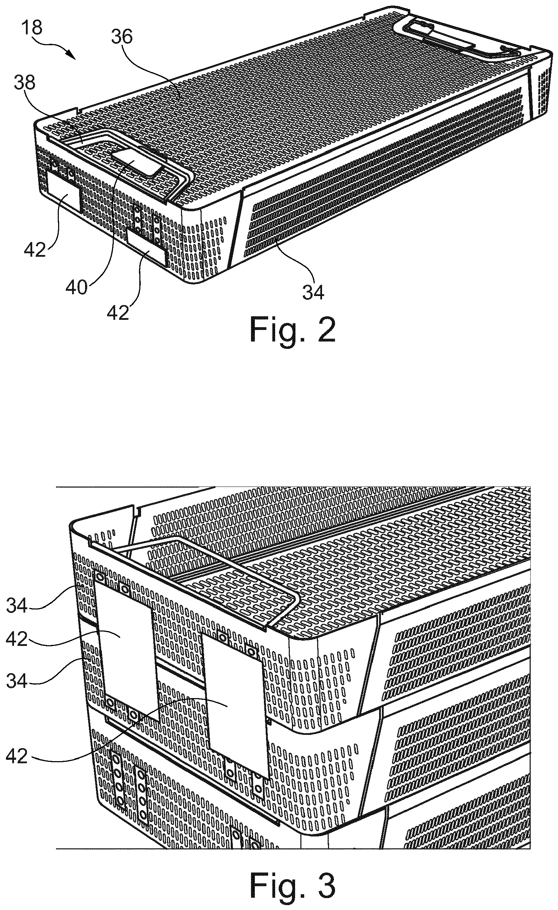

Figur 2 zeigt einen verschlossenen Bereitstellungsträger 18 zur Verwendung im Sterilgutkreislauf gemäß Figur 1, in perspektivischer Ansicht. Er hat eine quaderförmige Grundform und ist klassisch als Siebkorb 34 mit oberseitigem, abnehmbarem Siebdeckel 36 ausgestaltet. Der Siebkorb 34 hat an seinen Schmalseiten oberseitig je einen schwenkbaren Bügelgriff 38, der zum Festlegen des Siebdeckel 36 in Eingriff mit einem deckelseitigen Rastmechanismus 40 bringbar ist. Zu Nachverfolgung und Registrierung sind am Bereitstellungsträger 18 lesbare, auslesbare und insbesondere scanbare Identifikatoren 42 angebracht, vorzugsweise verrastet oder verclipst.

Figur 3 zeigt eine Vielzahl von übereinander gestapelten Bereitstellungsträgern 18, genauer gesagt übereinander gestapelter Siebkörbe 34 der Bereitstellungsträger 18 zur Verwendung im Sterilgutkreislauf gemäß Figur 1, in perspektivischer Ansicht. Dabei sind die oberen beiden Siebkörbe 34 über Identifikatoren 42 zu einem Siebtitel zusammengefasst. Hierzu sind die Identifikatoren 42 in Ausnehmungen jeweils des oberen und des unteren Siebkorbes eingerastet oder eingeclipst, sodass die beiden Siebkörbe durch die Identifikatoren 42 der Indikation zugeordnet sind und zudem mechanisch zu einer Einheit verbunden sind.

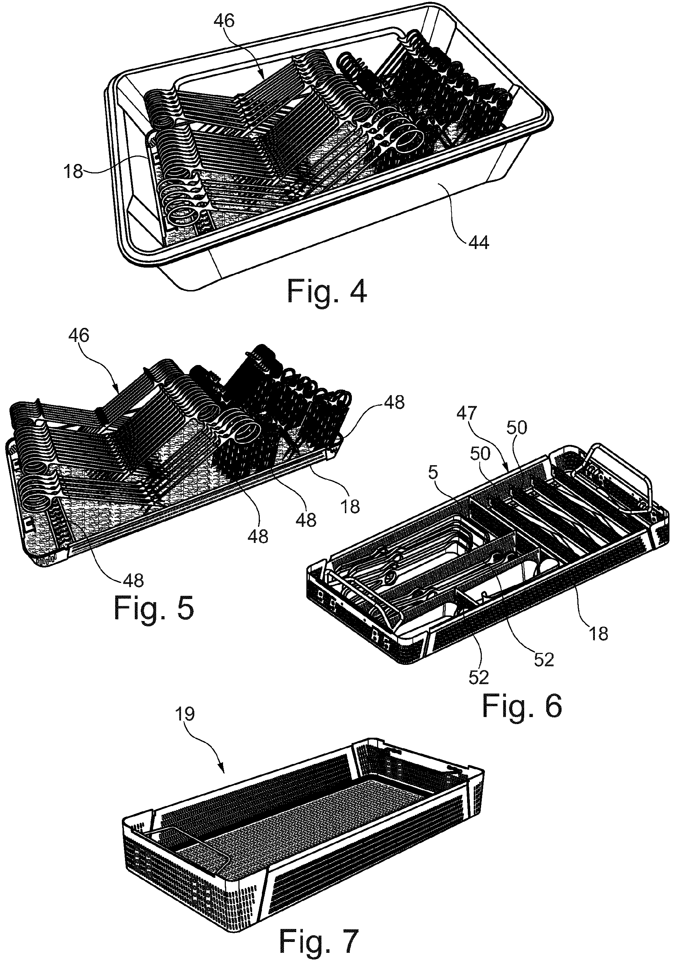

Figur 4 zeigt einen mit Ringgriff instrumenten 46 bestückten Bereitstellungsträger 18 in einer Wanne 44 eines Sterilgutcontainers 26, wie er sich beispielsweise an der Packstation 26 oder beim Öffnen des Sterilgutcontainers im OP darstellt, in perspektivischer Ansicht. Dabei ist der mit Ringgriffinstrumenten 46 bestückte, obere Bereitstellungsträger 18 auf einem mit starren, einteiligen Instrumenten bestückten Bereitstellungsträger gestapelt (nicht zu erkennen).

Figur 5 zeigt den von der Wanne 44 gemäß Figur 4 separierten, mit den Ringriffinstrumenten 46 bestückten Bereitstellungsträger 18. Letztgenannter ist als flacher Siebkorb ausgestaltet. Hierbei ist gut zu erkennen, dass die Ringriffinstrumente 46 gemäß der Offenbarung, jeweils an einem ihnen vorbestimmten Lagerplatz des Bereitstellungsträgers 18 angeordnet sind. Hierzu weist der Bereitstellungsträger 18 eine Anzahl unterschiedlicher, auf die Größen der Ringriffinstrumente 46 abgestimmter Ständer oder Böcke 48 auf, die Aufnahmen aufweisen, in die Hälse und Ringe der Ringriffinstrumente 46 eingehängt sind. Auf diese Weise ist der Bereitstellungsträger 18 mit den Ringriffinstrumenten 46 in deren wartungsfähiger Position bestückt. So können beispielsweise deren Gelenke geölt werden, ohne dass die Ringriffinstrumente 46 entnommen werden müssten (vergleiche entsprechende Varianten A - E der oben beschriebenen Wartung auf der Reinseite).

Figur 6 zeigt den von der Wanne 44 gemäß Figur 4 separierten, mit starren oder einteiligen Instrumenten 47 bestückten Bereitstellungsträger 18. Auch dieser ist als flacher Siebkorb ausgestaltet. Auch hier ist gut zu erkennen, dass die starren Instrumente 47 gemäß der Offenbarung jeweils an einem ihnen vorbestimmten Lagerplatz des Bereitstellungsträgers 18 angeordnet sind. Da aufgrund der starren Ausführung eine gezielte Wartung eines Gelenkes entfällt, reicht es aus, dass der Bereitstellungsträger 18 für die geordnete Bestückung mit den Instrumenten 47 in einzelne Bereitstellungsträgerabschnitte 50 mit vorzugsweise rechteckiger Grundfläche unterteilt ist. Die Bereitstellungsträgerabschnitte 50 sind vorzugsweise von flexibel am Boden und/oder an Seitenwänden des Bereitstellungsträgers 18 einrastbaren oder einclipsbaren Teilungselementen 52 oder durch an die Größe und Menge der Instrumente 47 angepasste Korbinserts definiert.

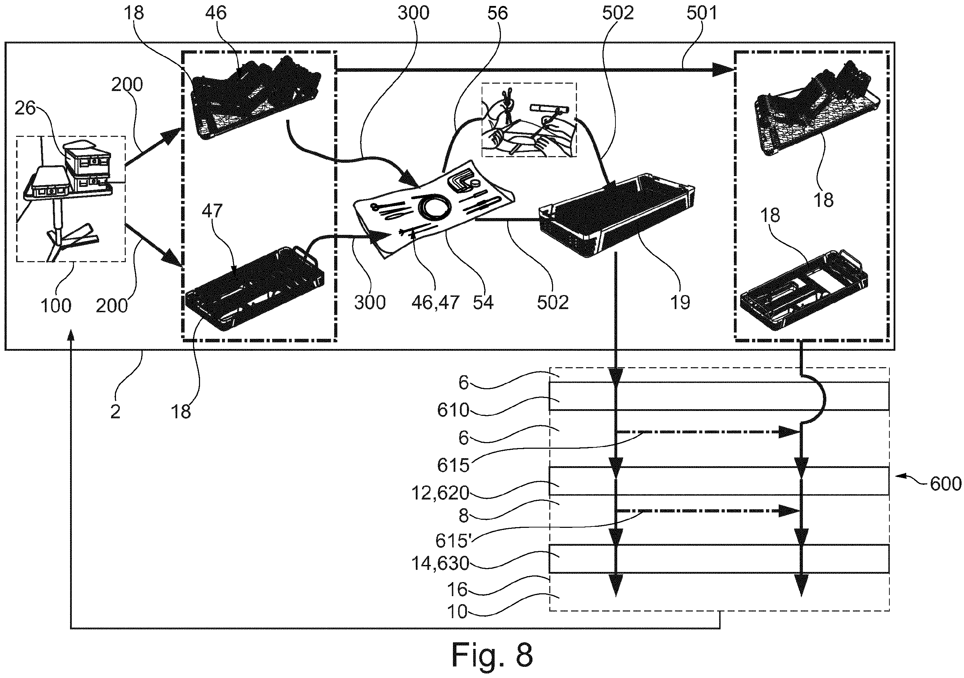

Figur 7 zeigt einen offenen Entsorgungsträger 19 zur Verwendung im Sterilgutkreislauf gemäß Figur 1 und im offenbarungsgemäße Verfahren gemäß der folgenden Figur 8, in perspektivischer Ansicht, der im Wesentlichen dem Bereitstellungsträger gemäß Figur 2 ohne den Siebdeckel 36 entspricht.

Fig. 8 zeigt das Verfahren zur Handhabe von aufbereitungsfähigem Sterilgut gemäß Figur 1, gemäß der vorliegenden Offenbarung, in detaillierterer Darstellung.

Zunächst erfolgt ein Schritt 100 "Bereitstellen des Sterilgutcontainers 26 mit den darin enthaltenen Instrumenten 46, 47" im OP. Dabei wird einer oder es werden mehrere der Indikation entsprechende Sterilgutcontainer 26 bereitgestellt.

Es folgt ein Schritt 200 "Separieren mehrerer mit den Instrumenten 46, 47 bestückter Bereitstellungsträger 18 vom Sterilgutcontainer 26".

Zum Eingriff erfolgt initial und fortlaufend der Schritt 300 "Entnehmen der Instrumente 46, 47 von den Bereitstellungsträgern 18", gemäß den Anforderungen der Indikation und des Operateurs. Dabei werden die Instrumente 46, 47 auf einer gesonderten Instrumentenablage 54 eines Instrumententischs platziert.

Während des Eingriffs werden die Instrumente 46, 47 je nach Bedarf verwendet, was durch einen zum Ort der medizinischen Versorgung weisenden Verwendungspfad 56 symbolisiert wird, oder sie werden weiterhin vorgehalten und verbleiben auf der Instrumentenablage 54.

Auch an dieser Stelle sei noch einmal - wie bereits weiter oben - darauf hingewiesen, dass der Eingriff und die Verwendung der Instrumente 46, 47 am Patienten nicht Teil des offenbarungsgemäßen Verfahrens sind und dementsprechend nicht erfindungsrelevant sind. Das wird in Figur 8 dadurch verdeutlicht wird, dass der dort schematisch dargestellte Eingriff und die dabei erfolgende Verwendung von Instrumenten gestrichelt-gerahmt dargestellt sind.

Während des möglichen Eingriffs, insbesondere am Ende des Eingriffs, werden die Instrumente 46, 47 in einem Verfahrensschritt 501, 502 "Entsorgen der entnommenen und der nicht entnommenen Instrumente 46, 47" entsorgt. Das betrifft alle Instrumente 46, 47, das heißt sowohl die vom Bereitstellungsträger 18 hin zum Instrumententisch 54 entnommenen, das heißt vorgehaltenen und möglicherweise verwendeten, Instrumente 46, 47, als auch die nicht vom Bereitstellungsträger 18 entnommenen, das heißt die auf dem Bereitstellungsträger 18 verbliebenen, Instrumente 46, 47.

Letztgenannte werden beim Schritt 501 "Entsorgen" offenbarungsgemäß unverändert in ihrer wartungsfähigen Position am jeweiligen Bereitstellungsträger 18 belassen. Die entnommenen, vorgehaltenen und die entnommenen, möglicherweise verwendeten Instrumente 46, 47 werden beim Schritt 502 "Entsorgung" offenbarungsgemäß in einem separat zu den Bereitstellungsträgern 18 im OP vorgesehenen Entsorgungsträger 19 platziert.

Im Anschluss an die Entsorgung 501, 502 werden die Bereitstellungsträger 18 und der gesonderte Entsorgungsträger 19 in die AEMP 4 verbracht, in welcher der Schritt 600 "Aufbereiten der entsorgten Instrumente 46, 47" - beginnend mit einer Vorreinigung 610 auf der Unreinseite 6, über eine Reinigung und Desinfektion 620 in den RDGs 12 hin zur Reinseite 8 und über eine Sterilisation 630 in den Sterilisatoren 14 (vgl. Figur 1) hin zur Sterilseite 10 - erfolgt.

Aufgrund der Verwendung getrennter Träger 18 und 19 ist es offenbarungsgemäß möglich, die manuelle Vorreinigung 610 allein auf die auf den Entsorgungsträger 19 entsorgten Instrumente 46, 47 anzuwenden, da nur diese aufgrund der zuvor geschilderten Entnahme und Ablage auf dem Instrumententisch 54, als benutzt klassifiziert sind. Die Bereitstellungsträger 18, und damit die darauf angeordneten ("nicht entnommenen") Instrumente 46, 47 werden an der Vorreinigung 610 vorbeigeführt, was in Figur 8 anhand des den Schritt 610 umgehenden Pfeiles symbolisiert wird. Dadurch entfällt der Schritt der "manuellen Vorreinigung" 610 für bis zu 70% der ursprünglich mit dem Schritt 200 "Separieren" bereitgestellten Instrumente 46, 47, was eine erhebliche Reduktion des Aufwandes in der AEMP 4 darstellt.

Grundlegend ist es möglich, dass die Breitstellungsträger 18 und der wenigstens eine Entsorgungsträger 19 den folgenden Schritt 620 "Reinigung und Desinfektion", beziehungsweise die RDGs 12, getrennt durchlaufen, sodass die RDGs 12 mit an die unterschiedliche Kontamination ("entnommenen Instrumente" versus "nicht entnommene Instrumente") angepassten Prozessparametern betrieben werden können.

Als Prozessparameter sind insbesondere eine kürzere Laufzeit der Bereitstellungsträger 18 in den RDGs 12 oder eine Applikation einer "sanfteren" Chemikalie oder einer geringeren Menge an Chemikalie auf die Bereitstellungsträger 18 in den RDGs12 zu nennen.

In einer Alternative zum getrennten Durchlaufen der "Reinigung und Desinfektion" werden in einem Schritt 615 die vorgereinigten Instrumente 46, 47 vom Entsorgungsträger 19 entnommen, den Bereitstellungsträgern 18 zugeführt und auf diesen in vorbestimmter Ordnung und Lage, insbesondere in kontroll- und wartungsfähiger Lage, platziert.

Diese Platzierung erfolgt insbesondere mittels der in Figur 5 und 6 beschriebenen Ständer oder Böcke 48 und der Bereitstellungsträgerabschnitte 50, je nachdem, ob es sich um mehrteilige, gelenkige Instrumente 46 oder starre, einteilige Instrumente 47 handelt.

Die Kontrolle und Wartung der Instrumente 46, 47 kann nach dem Schritt 610 "Vorreinigung" 610 noch vor Eintritt in die RDGs 12 und vor dem Schritt 620 "Reinigung und Desinfektion" erfolgen. Bevorzugt erfolgt die Kontrolle und Wartung der Instrumente 46, 47 erst nach dem Schritt 620 "Reinigung und Desinfektion".

Bei getrenntem Durchlauf der RDGs 12 entfällt der Schritt 615 auf der Unreinseite 6 und stattdessen werden die vorgereinigten und gereinigten und desinfizierten Instrumente 46, 47 im Schritt 615` vom Entsorgungsträger 19 ausgangsseitig der RDGs 12 entnommen, den Bereitstellungsträgern 18 zugeführt und auf diesen in vorbestimmter Ordnung und Lage, insbesondere in kontroll- und wartungsfähiger Lage, platziert.

Auf der Sterilseite 10 erfolgt wiederum die Freigabe 30 und in Folge die Kommissionierung und Lagerung 32 der Sterilgutcontainer 26, wie es bereits in Figur 1 erläutert wurde. Der Sterilgutkreislauf schließt sich, wenn diese Sterilgutcontainer 26 für eine weitere medizinische Versorgung im OP 2 erneut angefordert werden und im OP 2 bereitgestellt werden (Schritt "Bereitstellen" 100, siehe Beschreibung weiter oben).

Die vorliegende Offenbarung betrifft zusammenfassend ein Verfahren (oder ein Bereitstellungssystem) zur Bereitstellung, Entsorgung und Aufbereitung von medizinischem Sterilgut, insbesondere von chirurgischen Instrumenten, das die getrennte Entsorgung von kontaminiertem entnommenem/benutztem und nicht entnommenem/unbenutztem Sterilgut beinhaltet, das am Ort der medizinischen Versorgung einem Sterilbarrieresystem entnommen wurde. Die getrennte Entsorgung erfolgt dabei in Abhängigkeit von der potentiellen Kontamination des Sterilgutes am Ort der medizinischen Versorgung. In Folge können Teilprozesse der Aufbereitung in Abhängigkeit der unterschiedlichen, potentiellen Kontamination der beiden Gruppen von Sterilgut unterschiedlich appliziert werden, was Potential zur Effizienzsteigerung im Sterilgutkreislauf birgt.

Die Begriffe "manuelle Reinigung" oder "teilmanuelle Reinigung" beschränken das Verfahren nicht darauf, dass diese Schritte von einer Person durchgeführt werden müssen. Vielmehr dienen sie lediglich der Abgrenzung von einer Behandlung aller Instrumente auf einem Siebträger von der Behandlung einzelner Instrumente von einem Siebträger. Die manuelle oder teilmanuelle Reinigung kann also ebenfalls maschinell erfolgen, beispielsweise mittels eines robotischen Systems.

### Bezugszeichenliste

- 1: Sterilgutkreislauf
- 2: OP
- 4: Aufbereitungseinheit für medizinische Produkte, AEMP
- 6: Unreinseite AEMP
- 8: Reinseite AEMP
- 10: Sterilseite AEMP
- 12: Reinigungs- und Desinfektionsgerät, RDG
- 14: Sterilisator
- 16: Wareneingang AEMP
- 18: Bereitstellungsträger
- 19: Entsorgungsträger
- 20: Beladung
- 22: Freigabe
- 24: Bestückungsplatz
- 26: Sterilbarrieresystem
- 28: Beladung
- 30: Freigabe
- 32: Lagerung
- 34: Siebkorb
- 36: Siebdeckel
- 38: Bügelgriff
- 40: Rastmechanismus
- 42: Identifikator
- 44: Wanne
- 46: Instrument mehrteilig
- 47: Instrument einteilig
- 48: Ständer
- 50: Bereitstellungsträgerabschnitt
- 52: Teilungselement
- 54: Instrumentenablage
- 56: Verwendungspfad
- 100: Schritt Bereitstellen
- 200: Schritt Separieren
- 300: Schritt Entnehmen
- 501, 502: Schritt Entsorgen
- 610: Schritt Vorreinigen
- 615; 615`: Schritt Bestücken
- 620: Schritt Reinigen und Desinfizieren
- 630: Schritt Sterilisieren

## Patentansprüche

1. Verfahren zum Handhaben von aufbereitungsfähigem, medizinischem Sterilgut (46, 47), mit Schritten,
- "Bereitstellen eines Sterilbarrieresystems (26) mit darin enthaltenem Sterilgut (46, 47)" (100) an einem Ort (2) der medizinischen Versorgung, vorzugsweise in einem OP,
- "Separieren wenigstens eines mit dem Sterilgut (46, 47) bestückten Bereitstellungsträgers (18) vom Sterilbarrieresystem (26)" (200),
- "Bereitstellen eines separat zum wenigstens einen Bereitstellungsträger (18) vorgesehenen Entsorgungsträgers (19)",
- "Entnehmen zumindest von Sterilgut (46, 47) vom Bereitstellungsträger (18)" (300),
- "Platzieren des entnommenen Sterilgutes (46, 47) auf dem wenigstens einen Entsorgungsträger (19)",
- "Belassen des nicht entnommenen Sterilgutes auf dem wenigstens einen Bereitstellungsträger (18)" (501),
- "Entsorgen des entnommenen und des nicht entnommenen Sterilgutes" (501, 502), insbesondere im Anschluss an eine medizinische Versorgung, und in Folge
- "Aufbereiten des entsorgten Sterilgutes (46, 47)", zumindest teilweise mittels einer Aufbereitungseinheit für Medizinprodukte (4), wobei
- der Schritt "Aufbereiten des entsorgten Sterilgutes (46, 47)" einen manuellen oder zumindest teilmanuellen Aufbereitungsschritt (610), vorzugsweise einen Schritt "Vorreinigung", der auf das Sterilgut (46, 47) des Entsorgungsträgers (19) angewendet wird und nicht auf das Sterilgut (46, 47) des Bereitstellungsträgers (18), und
- einen Schritt "Reinigen und Desinfizieren" (620) mittels wenigstens einer Reinigungs- und Desinfektionseinrichtung (12) beinhaltet, wobei dieser Schritt keine manuelle oder teilmanuelle Bearbeitung erfordert.

2. Verfahren zumindest nach Anspruch 1 wobei der Schritt "Entsorgen" einen Schritt "Beschicken der Aufbereitungseinheit (4) mit dem wenigstens einen Bereitstellungsträger (18) und dem wenigstens einen Entsorgungsträger (19)" beinhaltet.

3. Verfahren nach einem der vorherigen Ansprüche, wobei der wenigstens eine Bereitstellungsträger (18) im Sterilbarrieresystem (26) mit wartungsbedürftigem und/oder mehrteiligem Sterilgut (46) bestückt bereitgestellt wird, das in wartungsfähiger Position und/oder Lage am Bereitstellungsträger (18) angeordnet ist.

4. Verfahren nach einem der vorherigen Ansprüche, wobei der wenigstens eine Bereitstellungsträger (18) im Sterilbarrieresystem (26) mit wartungsfreiem und/oder einteiligem Sterilgut (47) bestückt bereitgestellt wird.

5. Verfahren nach einem der vorherigen Ansprüche, wobei der wenigstens eine Bereitstellungsträger (18) mit dem Sterilgut (46, 47) in vorbestimmter Ordnung bestückt bereitgestellt wird.

6. Verfahren nach einem der vorhergehenden Ansprüche, wobei der Schritt "Aufbereiten des entsorgten Sterilgutes (46, 47)" einen Schritt "Sterilisieren" (630) mittels einer Sterilisationseinrichtung (14) beinhaltet.

7. Verfahren nach einem der vorhergehenden Ansprüche, wobei der Schritt "Aufbereiten des entsorgten Sterilgutes (46, 47)" wenigstens einen der Schritte "Kontrollieren des Sterilgutes" und "Warten des Sterilgutes, insbesondere von dessen Gelenken", beinhaltet.

8. Verfahren nach einem der vorhergehenden Ansprüche, wobei der Schritt "Aufbereiten des entsorgten Sterilgutes (46, 47)" einen Schritt "Bestücken des wenigstens einen Bereitstellungsträgers (18) mit dem vorgereinigten, entnommenen Sterilgut" (615; 615`) beinhaltet.

9. Verfahren nach Anspruch 4 und nach Anspruch 7 oder 8, wobei der jeweilige Schritt "Kontrollieren", "Warten" oder "Bestücken (615; 615`)" frühestens nach dem manuellen oder zumindest teilmanuellen Aufbereitungsschritt (610) erfolgt, bevorzugt nach dem Schritt "Reinigung und Desinfektion" (620).

10. Verfahren nach einem der vorherigen Ansprüche, wobei wenigstens einer der Schritte "Reinigung und Desinfektion" und "Sterilisation", oder beide, auf das entnommene Sterilgut und auf das nicht entnommene Sterilgut identisch angewendet wird oder werden, oder wobei wenigstens einer der Schritte "Reinigung und Desinfektion" (620) und "Sterilisation" (630), oder beide, auf das entnommene Sterilgut und auf das nicht entnommene Sterilgut unterschiedlich in Bezug auf wenigstens einen Prozessparameter des Schrittes angewendet wird oder werden.

11. Verfahren nach einem der vorherigen Ansprüche, wobei der Schritt "Bestücken des Bereitstellungsträgers mit dem entnommenen Sterilgut" vor der Reinigung und Desinfektion erfolgt (615) oder wobei er spätestens vor der Sterilisation erfolgt (615`).

12. Verfahren nach einem der vorhergehenden Ansprüche, wobei am Bereitstellungsträger (18) ein lesbarer, auslesbarer oder scanbarer Identifikator (42) angeordnet ist, und wobei dem Bereitstellungsträger (18) ein dem Identifikator (42) zugeordneter lesbarer, auslesbarer oder scanbarer Identifikator beigelegt ist, der nach dem Schritt "Separieren" (200) lesbar am Entsorgungsträger (19) angeordnet wird.
